(19) **Europäisches Patentamt**
**European Patent Office**
**Office européen des brevets**

(11) **EP 2 136 753 B1**

(12) **EUROPÄISCHE PATENTSCHRIFT**

(45) Veröffentlichungstag und Bekanntmachung des Hinweises auf die Patenterteilung:
**25.05.2011 Patentblatt 2011/21**

(51) Int Cl.:
***A61F 13/15*** *(2006.01)*

(21) Anmeldenummer: 08734628.4

(22) Anmeldetag: **15.03.2008**

(86) Internationale Anmeldenummer:
**PCT/EP2008/002092**

(87) Internationale Veröffentlichungsnummer:
**WO 2008/125175 (23.10.2008 Gazette 2008/43)**

(54) **VERFAHREN ZUR HERSTELLUNG EINES ELASTISCHEN VLIESSTOFFVERBUNDMATERIALS**

METHOD FOR PRODUCING AN ELASTIC NON-WOVEN COMPOSITE MATERIAL

PROCÉDÉ DE PRODUCTION D'UN MATÉRIAU COMPOSITE NON-TISSÉ ÉLASTIQUE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **17.04.2007 DE 102007018377**

(43) Veröffentlichungstag der Anmeldung:
**30.12.2009 Patentblatt 2009/53**

(73) Patentinhaber: **Paul Hartmann AG**
**89522 Heidenheim (DE)**

(72) Erfinder: **MALOWANIEC, Krzysztof, Daniel**
**89522 Heidenheim (DE)**

(56) Entgegenhaltungen:
**WO-A-02/18125         WO-A-95/34264
US-A- 4 223 063       US-A- 5 219 633
US-A1- 2006 246 803**

**Beschreibung**

Die Erfindung betrifft ein Verfahren zur Herstellung eines elastischen Vliesstoffverbundmaterials nach Anspruch 1.

Absorbierende Einwegartikel wie Hygieneartikel in Pantform umfassen häufig elastische Komponenten, um die Passform des Hygieneartikels und damit den Tragekomfort zu verbessern.

Die elastischen Komponenten umfassen häufig eine Vielzahl von Elastifizierungsmitteln, häufig in Form von elastischen Fäden, die im vorgespannten Zustand mit im Wesentlichen unelastischen Chassismaterialien zumeist klebend verbunden werden. Dabei ist üblicherweise ein Hüftrandbereich vorzugsweise durchgehend in Umfangsrichtung elastifiziert. Auch im Vorderbereich und im Rückenbereich sind bei bekannten Windelhosen Elastifizierungsmittel vorgesehen. Desgleichen werden die Beinöffnungen umgebende bzw. die Beinöffnungen bildende Umfangsbereiche zumindest abschnittsweise elastisch ausgestaltet, damit dort ein weitgehend dichtendes Anliegen des Hygieneartikels an die Hautoberfläche des Benutzers gewährleistet ist, um das seitliche Austreten von Körperausscheidungen zu verhindern. Auch aufstehende Bündchenelemente, die neben den elastischen Beinöffnungen einen weiteren seitlichen Auslaufschutz bieten, werden bei bekannten Windelhosen bereits eingesetzt und sind (beispielsweise aus EP-1184017-A1, EP-1199058-A1, EP-1308148-A2) bekannt.

Es ist ferner bekannt, zwischen Hüftrand und Schrittbereich Vliesstoffverbundmaterialien zu verwenden mit im Wesentlichen in einer Querrichtung erstreckten Elastifizierungsmitteln in Form von elastischen Fäden, die im vorgespannten Zustand mit im Wesentlichen unelastischen Chassismaterialien zumeist klebend verbunden werden (stretchbonding).

Ein Verfahren zur Herstellung eines derartigen Verbundmaterials offenbart bereits die WO01/88245. Das Verfahren umfasst das Extrudieren der elastischen Filamente und das Verbinden der Filamente mit einer Vliesstoffbahn mittels eines Klebstoffes, wobei die elastischen Filamente in vorgespanntem Zustand mit der Vliesstoffbahn verbunden werden (stretch-bonding).

Nachteil dieses Verfahrens ist die Notwendigkeit der Verwendung großer Klebermengen zur Verbindung der elastischen Filamente mit der Vliesstoffbahn.

US 5 219 633 und US 4 223 063 sind zwei andere Dokumenten des Stands der Technik.

Es ist daher eine Aufgabe der Erfindung, ein Verfahren zur Herstellung eines elastischen Vliesstoffverbundmaterials mit elastischen Filamenten bereit zustellen, das diesen Nachteil vermeidet.

Diese Aufgabe wird bei dem erfindungsgemäßen Verfahren gelöst durch Zuführen und Fördern einer ersten Vliesstofflage in einer Längsrichtung; Zuführen einer Vielzahl elastischer Filamente; Verbinden der Vielzahl elastischer Filamente mit einer ersten Seite der Vliesstofflage zur Bildung eines Materialverbundes; Zumindest bereichsweises Überdehnen der ersten Vliesstofflage in der Längsrichtung durch Hindurchführen des Materialverbundes durch den Spalt eines ersten Paars profilierter, ineinander greifender Dehnwalzen, wodurch der Materialverbund in der Längsrichtung gestreckt wird, wobei die elastischen Filamente derart angeordnet werden, dass sie im Wesentlichen in der Längsrichtung orientiert sind, so dass sie höchstens 10 Schnittpunkte miteinander pro cm$^2$ aufweisen. Vorzugsweise werden die elastischen Filamente derart angeordnet, dass sie parallel zueinander in der Längsrichtung verlaufen.

Während im durch die WO01/88245 dokumentierten Stand der Technik die verfügbare Dehnung des Laminates durch das Stretch-Bonding, also das Verbinden der vorgespannten elastischen Filamente mit der Vliesstofflage gewährleistet wird, wird mit der vorliegenden Erfindung zumindest ein wesentlicher Teil der verfügbaren Dehnung dadurch erzielt, dass die Vliesstofflage im bereits verbundenen Materialverbund einem nachträglichen Überdehnen, das heißt einer permanenten Verformung unterzogen wird, während sich die elastischen Filamente während des Streckvorganges im Wesentlichen elastisch verformen. Das Überdehnen erfolgt erfindungsgemäß durch Hindurchführen des Materialverbundes durch den Spalt eines ersten Paars profilierter, ineinander greifender Dehnwalzen. Das Verbinden von elastischen Filamenten und der Vliesstofflage kann also zu einem Zeitpunkt stattfinden, zu dem die elastischen Filamente keiner signifikanten Vorspannung unterzogen sind. Dies hat den Vorteil, dass den Haltekräften zwischen der Vliesstofflage und den elastischen Filamenten im Zuge der Herstellung des Vliesstoffverbundmaterials weitaus weniger Kräfte entgegengesetzt werden und mithin weniger Klebstoff verwendet werden muss oder gar auf den Einsatz eines zusätzlichen Haftvermittlers ganz verzichtet werden kann. In einer bevorzugten Weiterbildung der Erfindung beträgt die Vorspannung sogar weniger als 1,5, insbesondere weniger als 1,3, weiter insbesondere weniger als 1,2, weiter insbesondere weniger als 1,1 und vorzugsweise weniger als 1,05. Hierbei bedeutet eine Vorspannung von 1,5, das Dehnen der elastischen Filamente um 50 %, also beispielsweise, das Dehnen eines 10 cm langen Filamentabschnittes auf 15 cm.

Ein weiterer Vorteil ist ein gegenüber dem Stretch-Bonding deutlich geringerer Materialeinsatz, da wegen des Überdehnens der Vliesstofflage nach der vorliegenden Erfindung bei gleichem Materialeinsatz die Ausbeute an Laufmetern elastischer Vliesstoffverbundmaterialbahn deutlich erhöht ist.

In einer bevorzugten Form des Verfahrens wird der Materialverbund beim Hindurchführen des Materialverbundes durch den Spalt eines ersten Paars profilierter, ineinander greifender Dehnwalzen in Längsrichtung um 35-300%, insbesondere um 50-200% gestreckt. Unter Dehnung oder Streckung wird hierbei das Verhältnis der Längenänderung zur Ausgangslänge verstanden. Ein Abschnitt einer Ausgangslänge von beispielsweise 100 cm, welcher auf eine Länge von 150 cm gedehnt wird, würde nach diesem Verständnis um 50% gedehnt werden.

Je nach Ausmaß der mit der Streckung verbundenen permanenten Dehnung der Vliesstofflage resultiert daraus ein Vliesstoffverbundmaterial mit einer in Längsrichtung verfügbaren Dehnung (Dehnbarkeit) von 35-300%, insbesondere 50-200%.

Unter verfügbarer Dehnung oder Dehnbarkeit wird das Verhältnis der möglichen Längenänderung zur Ausgangslänge eines Abschnittes des Vliesstoffverbundmaterials verstanden. Die mögliche Längenänderung wird hierbei nach einer wie folgt standardisierten Methode ermittelt:

Ein 50 mm breiter (Dimension in Querrichtung des Vliesstoffverbundmaterials) und ca. 150 mm langer (Dimension in Längsrichtung des Vliesstoffverbundmaterials) Abschnitt des Vliesstoffverbundmaterials wird spannungslos über 24 h bei 23°C und 50% relativer Luftfeuchtigkeit klimatisiert. Anschließend wird der Vliesstoffverbundmaterialabschnitt in eine horizontale Zugprüfeinrichtung nach EN ISO 527-1 (1996) eingespannt, wobei der Abstand für die Einspannvorrichtung, also die Ausgangslänge L0 100 mm beträgt. Hierbei wird der Abschnitt über seine gesamte Breite von 50 mm an einem Ende in die feststehende Klemmbacke fest eingespannt. An dem anderen Ende wird der Abschnitt über seine gesamte Breite von 50 mm in die bewegliche Klemmbacke der Zugprüfeinrichtung fest eingespannt und bis zum Erreichen einer Kraft von 10N belastet, wobei die Geschwindigkeit der beweglichen Klemmbacke 500 mm/min beträgt. Anschließend wird die gedehnte Länge L1 des Abschnittes in mm gemessen.

Die verfügbare Dehnung in % berechnet sich dann nach folgender Formel:

$$Verf\ddot{u}gbare Dehnung [\%] = \frac{L1[mm] - L0[mm]}{L0[mm]} \, x100\%$$

Sollte kein genügend großer Abschnitt des Vliesstoffverbundmaterials zur Verfügung stehen, wird die verfügbare Dehnung an entsprechend kleineren Abschnitten ermittelt. Hierbei ist die Belastung jeweils auf einen Wert von 2 N / 10 mm Abschnittsbreite einzustellen.

Nach einem weiteren Erfindungsgedanken kann der Materialverbund nach dem Hindurchführen des Materialverbundes durch den Spalt eines ersten Paars profilierter, ineinander greifender Dehnwalzen durch einen jeweiligen Spalt weiterer Paare, insbesondere zweier weiterer Paare, vorzugsweise eines weiteren Paares profilierter, ineinander greifender Dehnwalzen hindurchgeführt werden. Solchenfalls kann die Überdehnung stufenweise erfolgen. In Weiterbildung dieses Erfindungsgedankens wird der Materialverbund durch das Hindurchführen des Materialverbundes durch den Spalt des ersten Paars profilierter, ineinander greifender Dehnwalzen vorzugsweise stärker gedehnt als durch das Hindurchführen des Materialverbundes durch den jeweiligen Spalt jedes weiteren Paares profilierter, ineinander greifender Dehnwalzen.

Aus wirtschaftlichen Gründen ist es vorteilhaft, dass die Vliesstofflage oder die Vliesstofflagen aus unelastischem Material gebildet sind.

In einer ersten Alternative des erfindungsgemäßen Verfahrens ist vorgesehen, die elastischen Filamente in an sich bekannter Weise in einem separaten Herstellprozess, also offline zu fertigen, anschließend endlos auf Rollen zu wickeln und die elastischen Filamente im Zuge der Herstellung des Vliesstoffverbundmaterials von der Rolle abzuwickeln. Denkbar und besonders vorteilhaft ist hingegen, dass das Verfahren der Herstellung des Vliesstoffverbundmaterials das Extrudieren der elastischen Filamente mit umfasst. Solchenfalls würde man von einer inline-Fertigung der elastischen Filamente sprechen.

Wie bereits weiter oben angedeutet erweist es sich insbesondere aus Kostengründen als vorteilhaft, die elastischen Filamente direkt, also insbesondere ohne weiteren Haftvermittler mit der Vliesstofflage zu verbinden. Hierzu können die elastischen Filamente ein Polymer mit permanenter Haftkraft enthalten. Im Falle der inline-Fertigung der elastischen Filamente kann die dauerhafte Verbindung zwischen elastischen Filamenten und Vliesstofflage auch dadurch erfolgen, dass die elastischen Filamente kurz nach der Extrusion in noch nicht völlig erstarrtem Zustand mit der Vliesstofflage in Kontakt gebracht werden. Denkbar und vorteilhaft ist auch, die elastischen Filamente gemeinsam mit der Vliesstofflage durch einen Pressspalt, insbesondere einen beheizbaren Pressspalt eines Walzenpaars, insbesondere eines Kalanderwalzenpaares hindurchzuführen. Hierbei können die Vliesstofflage und die elastischen Filamente vollflächig, vorzugsweise aber diskontinuierlich durch ein Bindemuster, also ein Muster gebundener und nicht gebundener Bereiche, insbesondere ein Punktbindemuster thermisch, also durch thermisch erzeugte Schweißbindungen verbunden werden. Vorteilhafterweise ist das Bindemuster derart ausgeführt, dass jedes der elastischen Filamente durch mindestens einen Bindebereich, insbesondere mindestens zwei, weiter insbesondere mindestens drei Bindebereiche verläuft.

Im Zusammenhang mit dem Schritt des Streckens des Vliesstoffverbundmaterials in seiner Längsrichtung kann es sich als vorteilhaft erweisen, die Bahn des Vliesstoffverbundmaterials in Querrichtung zu fixieren, um einer Einschnürung der Bahn in Querrichtung, das heißt einer Verringerung der Erstreckung in der Querrichtung entgegenzuwirken.

Gleichwohl kann es vorteilhaft sein, gezielt ein geringes Maß an Einschnürung, insbesondere weniger als 50%, weiter insbesondere weniger als 30%, weiter insbesondere weniger als 20% und ganz besonderes weniger als 10% zuzulassen. Die Einschnürung in % wird hierbei nach folgender Formel berechnet:

$$Einschnürung[\%] = \frac{LQ0 - LQ1}{LQ0} \times 100\% \,,$$

wobei LQ0 die Länge der Bahn in Querrichtung vor dem Verfahrensschritt des Überdehnens ist und LQ1 die Länge der Bahn in Querrichtung nach dem Verfahrensschritt des Überdehnens ist.

Solchenfalls ist es möglich, das Vliesstoffverbundmaterial mit einer zumindest geringfügigen Dehnbarkeit auch in Querrichtung auszustatten.

In einer weiteren Ausführungsform umfasst das erfindungsgemäße Verfahren das Zuführen einer zweiten Vliesstofflage. Vorteilhafterweise wird diese derart zugeführt und angeordnet, dass die erste und die zweite Vliesstofflage die elastischen Filamente sandwichartig einschließen. Es erweist sich als vorteilhaft, wenn die elastischen Filamente gemeinsam mit der ersten und der zweiten Vliesstofflage durch einen Pressspalt, insbesondere einen beheizbaren Pressspalt eines Walzenpaars hindurchgeführt werden. Hierbei können die Vliesstofflagen vollflächig, vorzugsweise aber diskontinuierlich durch ein Bindemuster, also ein Muster gebundener und nicht gebundener Bereiche, insbesondere ein Punktbindemuster thermisch verbunden werden. Vorteilhafterweise ist das Bindemuster derart ausgeführt, dass jedes der elastischen Filamente durch mindestens einen Bindebereich, insbesondere mindestens zwei, weiter insbesondere mindestens drei Bindebereiche verläuft.

Die erste und/oder die zweite Vliesstofflage können in an sich bekannter Weise in einem separaten Herstellprozess, also offline nach einem der im Stand der Technik bekannten Verfahren, wie zum Beispiel Spinnvlies-, Kardenvlies-, Spunlacevlies-, oder Melblownvliesherstellverfahren gefertigt und anschließend endlos auf Rollen gewickelt werden, um die vorgefertigten Vliesstofflagen dann im Zuge der Herstellung des elastischen Vliesstoffverbundmaterials von der Rolle abzuwickeln.

Die erste Vliesstofflage und vorzugsweise auch die zweite Vliesstofflage - falls eine zweite Vliesstofflage vorgesehen ist - weist vorzugsweise ein Flächengewicht von 5-30 g/m$^2$, insbesondere von 8-25 g/m$^2$, weiter insbesondere von 10-22 g/m$^2$, ganz besonders von 12-18 g/m$^2$ auf.

Die erste und/oder die zweite Vliesstofflage sind insbesondere in Längsrichtung und vorzugsweise auch in Querrichtung im Wesentlichen unelastisch. Dies trägt dazu bei, Kosten zu sparen und die weiter unten beschriebene zweiphasige Dehnungscharakteristik möglich machen zu können. Unter einer unelastischen Vliesstofflage wird im Rahmen der vorliegenden Erfindung ein Vliesstoffmaterial verstanden, das nach einer einmaligen Dehnung eines 25 mm breiten Materialstreifens um 30 % seiner Ausgangslänge bei einer Dehngeschwindigkeit von 500 mm/min entweder reißt oder bei anschließender sofortiger Entlastung eine permanente Dehnung von mindestens 7,5 % aufweist. Dies bedeutet, dass beispielsweise ein 100 mm langer Materialstreifen, der auf eine Länge von 130 mm gedehnt wurde, nach Entlastung eine Länge von mindestens 107,5 cm aufweist.

In Weiterbildung der Erfindung wird ferner vorgeschlagen, das Strecken des Vliesstoftmaterials derart auszuführen, dass erste Bereiche der Vliesstoftlage stärker überdehnt werden als zweite Bereiche der Vliesstofflage. Insbesondere vorteilhaft ist, die zweiten Bereiche im Wesentlichen gar nicht zu überdehnen. Vorzugsweise sind die ersten und zweiten Bereiche dann alternierend, quer zu der Längsrichtung der Bahn des Vliesstoffverbundmaterials angeordnet. Dies hat zur Folge, dass die Dehnungscharakteristik des Vliesstoffverbundmaterials in Gebrauch durch zwei Phasen gekennzeichnet ist. Die ersten stärker überdehnten Bereiche setzen dem Dehnen in Längsrichtung zunächst einen ersten, geringen Widerstand entgegen. Nach maximaler Ausdehnung der stark überdehnten Bereiche erfordert das weitere Dehnen der weniger stark oder gar nicht überdehnten Bereiche dann eine höhere Kraft, welche deutlich über den Kräften liegt, die bei normalem Gebrauch zu erwarten sind. Diese Zweiphasigkeit gibt dem Anwender einen Hinweis auf die maximale Dehnbarkeit des Materials, noch lange bevor das Material der Gefahr des Zerreißens ausgesetzt wird.

In einer weiteren vorteilhaften Ausführung der Erfindung beträgt die Stärke der elastischen Filamente 4-700 dtex, insbesondere 50-300 dtex und weiter insbesondere 100-200 dtex. In Weiterbildung dieses Erfindungsgedankens enthalten die erste und vorzugsweise auch die zweite Vliesstofflage Fasern mit einem ersten Titer, gemessen in dtex, während die elastischen Filamente einen zweiten Titer aufweisen, wobei der zweite Titer größer ist, vorzugsweise um bis zu 100% größer, besonders bevorzugt um bis zu 200% größer, insbesondere um bis zu 500% größer, vorzugsweise jedoch um höchstens 5000%, besonders bevorzugt um höchstens 4000 % und ganz besonders bevorzugt um höchstens 3000% größer ist als der erste Titer. Der Anteil des Gewichts der elastischen Filamente am Gesamtgewicht des Vliesstoffverbundmaterials beträgt dabei vorzugsweise weniger als 40 %, insbesondere weniger als 30 %, weiter insbesondere weniger als 20 %, weiter insbesondere weniger als 15 %, vorzugsweise aber mehr als 5%.

Die elastischen Filamente enthalten oder bestehen vorzugsweise aus einem thermoplastischen Polymer insbesondere einem extrudierbaren Polymer ausgewählt aus der Gruppe Polyurethane, elastische Polyester, elastische Polyamide, elastische Polyolefine oder elastische Blockcopolymere. Bevorzugte extrudierbare elastische Polymere offenbaren die WO01/88245 und die WO06/124092, die diesbezüglich vollumfänglich zum Offenbarungsgehalt der vorliegenden Erfindung gemacht werden.

Der Abstand der elastischen Filamente voneinander in Querrichtung beträgt vorzugsweise 0,5-10mm, besonders bevorzugt 1-5 mm, insbesondere 1,2-3,5 mm.

Die vorliegende Erfindung betrifft außerdem ein Vliesstoffverbundmaterial herstellbar oder hergestellt nach dem Verfahren wie zuvor gekennzeichnet. Vorzugsweise umfasst das Vliesstoffverbundmaterial mit einer Längsrichtung und einer senkrecht dazu verlaufenden Querrichtung eine zumindest bereichsweise überdehnte erste Vliesstofflage mit einer ersten Seite und einer zweiten Seite, sowie eine Vielzahl im Wesentlichen in der Längsrichtung orientierter elastischer Filamente, die mit der ersten Seite der Vliesstofflage verbunden sind, wobei die elastischen Filamente derart angeordnet sind, dass sie höchstens 10 Schnittpunkte miteinander pro cm$^2$ aufweisen, wobei das Vliesstoffverbundmaterial in der Längsrichtung vorzugsweise eine Dehnbarkeit von 35-300% aufweist. Insbesondere sind die elastischen Filamente derart konsequent in der Längsrichtung ausgerichtet, dass sie höchstens 8, insbesondere höchstens 5, insbesondere höchstens 3, weiter insbesondere höchstens 1 und ganz besonders gar keinen, also 0 Schnittpunkte pro cm$^2$ Vliesstoffverbundmaterial miteinander aufweisen. Vorzugsweise verlaufen die elastischen Filamente in der Längsrichtung parallel zu einander.

Außerdem ist ein wegwerfbarer absorbierender Hygieneartikel in Pantform mit einem eine Hüftöffnung bildenden in Umfangsrichtung durchgehend geschlossenen Hüftrand und mit Beinöffnungen, und mit Längsseitenrandabschnitte aufweisendem Vorderteil und Rückenteil, wobei Vorderteil und Rückenteil voneinander in Längsrichtung des Hygieneartikels beabstandet sind, und mit einem das Vorderteil und Rückenteil in Längsrichtung des Hygieneartikels verbindenden Absorptionsteil, wobei der in Umfangsrichtung durchgehend geschlossene Hüftrand und die Beinöffnungen gebildet sind, indem Längsseitenrandabschnitte eines Vorderteils und eines Rückenteils herstellerseitig miteinander verbunden sind, und wobei das Vorderteil und/oder das Rückenteil ein erfindungsgemäßes Vliesstoffverbundmaterial enthalten oder daraus bestehen und wobei die Längsrichtung des Vliesstoffverbundmaterials im Wesentlichen senkrecht zu einer Längsrichtung des Hygieneartikels verläuft Gegenstand der vorliegenden Erfindung. Insbesondere beträgt die Erstreckung des Vliesstoffverbundmaterials des Vorderteils und/oder des Rückenteils in Längsrichtung des Hygieneartikels 8-50 cm, insbesondere 10-40 cm und weiter insbesondere 12- 30 cm.

Die Wasserdampfdurchlässigkeit des Vliesstoffverbundmaterials beträgt insbesondere wenigstens 300g/m$^2$/24h, weiter insbesondere wenigstens 1000g/m$^2$/24h, weiter insbesondere wenigstens 2000g/m$^2$/24h, weiter insbesondere wenigstens 3000g/m$^2$/24h, weiter insbesondere wenigstens 4000g/m$^2$/24h, weiter insbesondere höchstens 6000g/m$^2$/24h gemessen nach DIN 53 122-1 (Ausgabe: 2001-08).

Weitere Merkmale, Einzelheiten und Vorteile der Erfindung ergeben sich aus den beigefügten Patentansprüchen und aus der zeichnerischen Darstellung und nachfolgenden Beschreibung bevorzugter Ausführungsformen der Erfindung. In der Zeichnung zeigt:

Figur 1 in schematisierter Form eine Seitenansicht einer Vorrichtung für den Betrieb eines erfindungsgemäßen Verfahrens

Figur 2 eine Schnittansicht entlang einer Linie A-A der Figur 1

Figur 3 eine Teilansicht eines Querschnittes der Figur 5 entlang der Linie B-B

Figur 4 eine vergrößerte Darstellung des Eingriffsbereich eines Dehnwalzenpaares

Figur 5 einen Teilabschnitt eines Materialverbundes in der Aufsicht

Figur 6 einen Querschnitt eines Teilabschnittes eines Materialverbundes entlang einer Linie B-B

Figur 7 einen Teilabschnitt eines elastischen Vliesstoffverbundmaterials in der Aufsicht im entspannten Zustand

Figur 8 einen Teilabschnitt eines elastischen Vliesstoffverbundmaterials in gedehntem Zustand

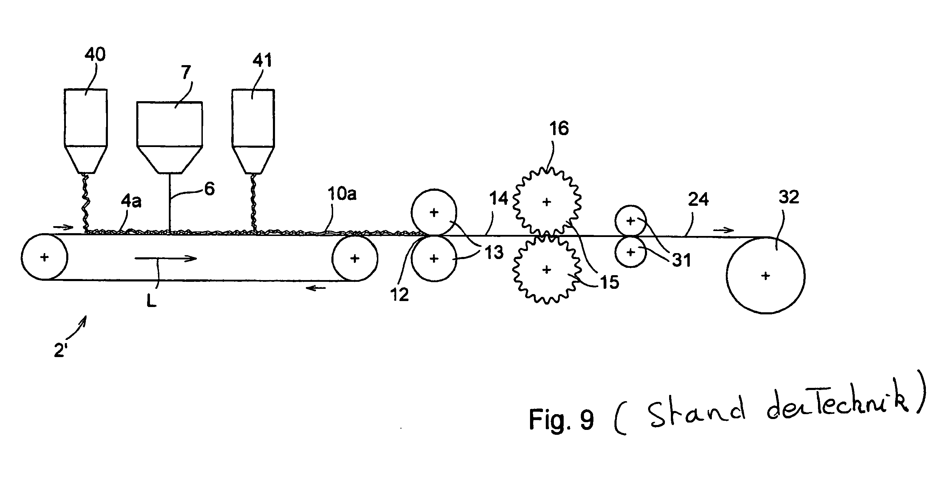

Figur 9 in schematisierter Form eine Seitenansicht einer weiteren Vorrichtung für den Betrieb eine Verfahrens des Stands der Technik

Figur 10 eine Draufsicht auf eine erste Ausführungsform eines pantförmigen Hygieneartikels im flachgelegten Zustand vor der herstellerseitigen Verbindung von Längsseitenrandbereichen

Figur 11    eine perspektivische Ansicht eines pantförmigen Hygieneartikels nach der herstellerseitigen Verbindung von Längsseitenrandbereichen

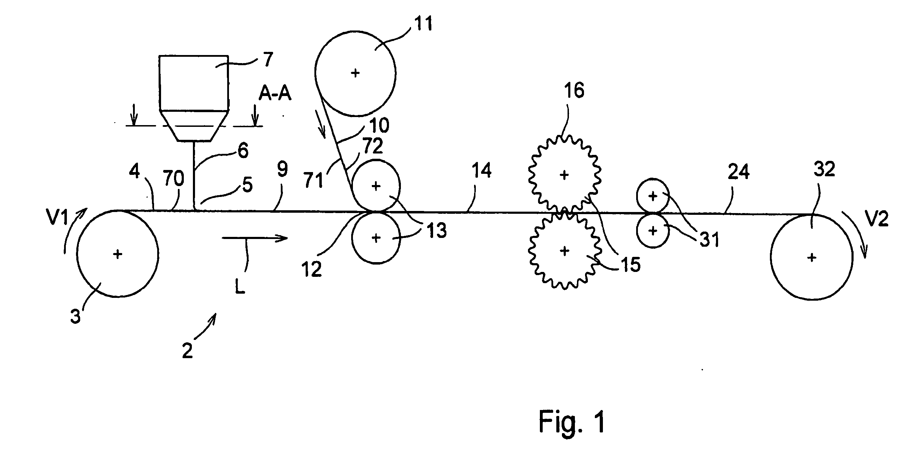

Figur 1 zeigt schematisch eine insgesamt mit dem Bezugszeichen 2 bezeichnete Vorrichtung zum Betreiben eines erfindungsgemäßen Verfahrens zur Herstellung eines elastischen Vliesstoffverbundmaterials. Von einer Rolle 3 wird eine erste bereits vorgefertigte unelastische Vlieslage 4, insbesondere eine PP- Spinnvlieslage mit einem Flächengewicht von 18 g/m$^2$ mit einer Geschwindigkeit v1 abgerollt und in Längsrichtung L einem Kontaktbereich 5 zugeführt, in dem die elastischen Filamente 6 auf eine erste Seite 70 der ersten Vlieslage 4 im Wesentlichen spannungsfrei abgelegt werden. Die elastischen Filamente 6 werden im dargestellten Fall inline durch einen Extruder 7 erzeugt. Denkbar wäre natürlich auch, die elastischen Filamente 6 offline zu fertigen und dem Vliesstoffverbundmaterialherstellungsprozess als vorgefertigtes Material in an sich bekannter Weise zum Beispiel von der Rolle endlos zuzuführen.

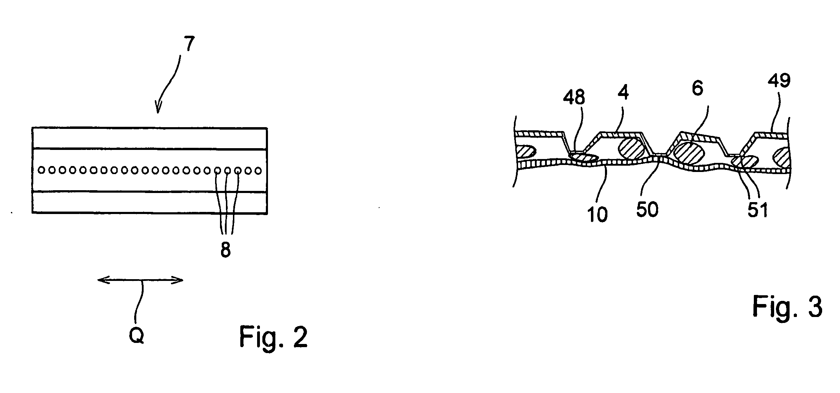

Figur 2 zeigt schematisch, nicht maßstabsgerecht, den Kopf des Extruders 7 im Querschnitt entlang einer Linie A-A. Erkennbar sind die Austrittsdüsen 8 für die elastischen Filamente 6. Der Abstand der Austrittsdüsen beträgt etwa 3 mm.

Der Kontakt der elastischen Filamente 6 mit der Vlieslage 4 im Kontaktbereich 5 erfolgt vorzugsweise zu einem Zeitpunkt, zu dem die extrudierten elastischen Filamente 6 noch nicht völlig erhärtet sind und somit in noch angeschmolzenem, mithin noch klebrigem Zustand.

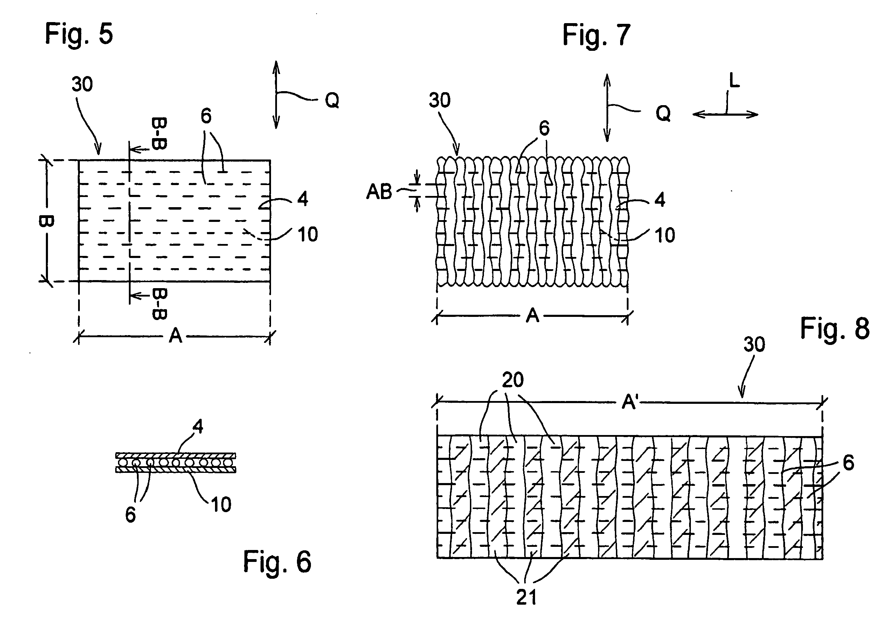

Das aus der ersten Vlieslage 4 und den elastischen Filamenten 6 bestehenden Vorlaminat 9 wird im dargestellten Fall beschichtet mit einer weiteren, zweiten unelastischen Vlieslage 10 mit einer ersten Seite 72 und einer zweiten Seite 71, insbesondere eine PP-Spinnvlieslage mit einem Flächengewicht von 18 g/m$^2$, welche von einer Rolle 11 abgerollt wird. Das Zusammenführen von Vorlaminat 9 und zweiter Vlieslage 10 erfolgt unmittelbar vor einem aus einem Kalanderwalzenpaar 13 gebildeten Pressspalt 12, derart, dass die zweite Seite 71 der zweiten Vlieslage 10 der ersten Seite 70 der ersten Vlieslage 4 zugewandt ist. Das Kalanderwalzenpaar 13 umfasst eine erste glatte, sowie eine mit inselförmigen Erhebungen ausgeführte zweite, insbesondere beheizte Walze, so dass durch das Hindurchführen durch den Pressspalt Vorlaminat 9 und zweite Vlieslage 10 mittels Punktbindemuster, also der Bildung von punktartigen gebundenen Bereichen 48 und diese umgebenden ungebundenen Bereichen 49, dauerhaft verbunden werden können. Hierbei werden sowohl erste Schmelzbindungen 50 direkt zwischen erster und zweiter Vlieslage 4, 10 als auch zweite Schmelzbindungen 51 zwischen erster und/oder zweiter Vlieslage 4, 10 und den elastischen Filamenten 6 erzeugt. Dabei sind die Vlieslagen (4, 10) so angeordnet, dass die elastischen Filamente 6 zwischen der ersten Seite 70 der ersten Vlieslage 4 und der zweiten Seite 71 der zweiten Vlieslage 10 positioniert sind. Figur 3 veranschaulicht das beschriebene Punktbindemuster in einer vergrößerten Teilansicht eines Querschnittes entlang einer Linie B-B der Figur 5.

Denkbar wäre auch anstelle des thermischen Verbindens durch das beschriebene Punktbindemuster, die Vlieslagen 4, 10 und die elastischen Filamente 6 durch einen Haftvermittler, insbesondere durch geringe Mengen eines Hotmeltklebstoffes zu verbinden. Dieser würde vorzugsweise über an sich bekannte Module wie Hotmeltsprüh- oder schlitzkopfauftragsvorrichtungen insbesondere auf die erste Seite 70 der ersten Vlieslage 4, jedenfalls vor dem Kalanderwalzenpaar 13 aufgetragen werden. Das Kalanderwalzenpaar 13 könnte solchenfalls durch zwei glatte Walzen gebildet sein, welche die Vlieslagen 4, 10 und die elastischen Filamente 6 vollflächig zusammendrücken und so die Verbundhaftung herbeiführen.

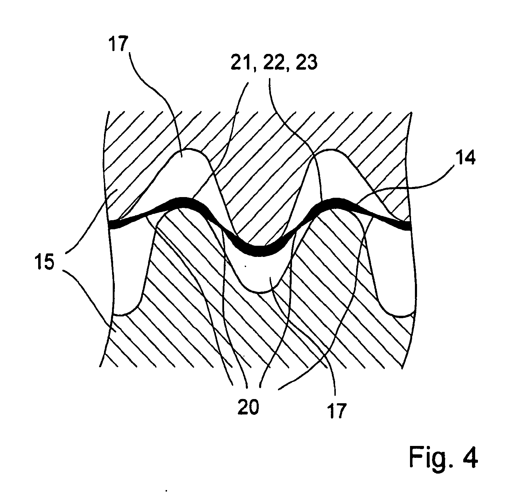

Im weiteren Verlauf wird der so gebildete Materialverbund 14 in den Spalt eines Dehnwalzenpaares 15 geführt. Figur 4 zeigt in einer vergrößerten Darstellung den Eingriffsbereich des Dehnwalzenpaares 15. Die Walzen dieses Dehnwalzenpaares 15 weisen eine gerillte Oberfläche 16 auf, wobei die Rillen 17 quer zur Längsrichtung L verlaufen. Die Rillengeometrie der Dehnwalzen ist derart aufeinander abgestimmt, dass diese berührungslos ineinander greifen können, wobei das Ausmaß des Ineinandergreifens und damit das Ausmaß das Streckens des Materialverbundes 14 regelbar ist. Die Geometrie der Rillen kann wie in Figur 4 dargestellt wellenförmig oder auch eckig gezähnt sein oder andere denkbare Konfigurationen annehmen. Wie Figur 4 veranschaulicht erfolgt das Strecken des Materialverbundes 14 derart, dass erste Bereiche 20 zwischen den Auflageabschnitten 22 des Materialverbundes 14 auf den Rillenbergen 23 stärker gedehnt werden als zweite Bereiche 21, die mit den Auflageabschnitten 22 korrespondieren. Die Streckung des Materialverbundes 14 resultiert in einer Überdehnung der Vlieslagen 4, 10, da die Vlieslagen 4, 10 in Längsrichtung im Wesentlichen unelastisch sind. Dies bedeutet, dass die Vlieslagen 4, 10 eine permanente, nicht elastische Verformung erfahren. Die elastischen Filamente hingegen werden beim Strecken des Materialverbundes 14 lediglich elastisch verformt. Somit lässt sich das Vliesstoffverbundmaterial 24 nach dem Streckprozess in Längsrichtung L elastisch verformen, wobei das Ausmaß der verfügbaren Dehnung in etwa der vorangegangenen Streckung entspricht.

Zur weiteren Veranschaulichung zeigt Figur 5 in der Aufsicht einen Teilabschnitt 30 des Materialverbundes 14 unmittelbar vor dem Hindurchführen durch den Spalt des Dehnwalzenpaares 15 mit der Ausgangslänge A = 100 cm und der Breite B = 60 cm. Die elastischen Filamente weisen eine Faserstärke von 100 dtex auf. Die Fasern beider Vlieslagen weisen eine Faserstärke von 4 dtex auf. Im schematisch illustrierten Querschnitt entlang B-B (Figur 6) sind die beiden Vlieslagen 4, 10 sowie die sandwichartig dazwischen im Wesentlichen spannungslos angeordneten elastischen Filamente 6 erkennbar. Auf eine Darstellung des Punktbindemuster wurde in Figur 6 verzichtet. Das Punktbindemuster im Detail zeigt Figur 3.

Figur 7 zeigt diesen Teilabschnitt 30 unmittelbar nach dem Hindurchführen durch den Spalt des Dehnwalzenpaares 15, wodurch zunächst eine Streckung des Teilabschnittes um 100 %, also auf eine Länge von 200 cm erfolgt. Hierbei sind erste Bereiche 20 der Vlieslage 4, 10 stärker überdehnt worden als zweite Bereiche 21 der Vlieslagen 4, 10. Erste und zweite Bereiche 20, 21 sind alternierend streifenförmig quer zu der Längsrichtung L angeordnet, wie in der weiter unten beschriebenen Figur 8 zu erkennen ist. Die Rückstellkräfte der mit den Vliesstofflagen 4, 10 verbundenen elastisch verformten elastischen Filamente 6 führen dazu, dass der Teilabschnitt 30 des Vliesverbundmaterials 24 sich wieder bis zur Ausgangslänge A = 100 cm zusammenzieht. Da die Streckung der Vlieslagen 4, 10 um 100% eine permanente Dehnung (Überdehnung) der Vlieslagen um ebenfalls 100% zur Folge hat, führt das gleichsam überschüssige Vlieslagenmaterial wie in Figur 7 erkennbar zur Fältelung/Raffung des Vlieslagen 4, 10. Der Abstand AB zwischen den im Wesentlichen parallel zueinander angeordneten elastischen Filamenten beträgt in Querrichtung Q 3mm. Die elastischen Filamente 6 weisen somit 0 Schnittpunkte miteinander pro $cm^2$ auf. Das Flächengewicht der Vlieslagen 4, 10 beträgt jeweils 18 g/$m^2$, Der Gewichtsanteil der elastischen Filamente 6 am Gesamtgewicht des Vliesstoffverbundmaterials 24 beträgt 8,5%.

Der Teilabschnitt 30 des fertigen Vliesverbundmaterials 24 lässt sich nun in Längsrichtung L auf eine Länge von 200 cm, mithin um 100% elastisch dehnen (Figur 8). Somit ist mit dem vorliegenden Verfahren im Vergleich zum bekannten "Stretch-Bonding" eine wesentliche Materialeinsparung von bis zu 100% möglich, da beim "Stretch-Bonding" ein auf 200 cm ausdehnbarer Teilabschnitt die Verwendung ebenfalls 200 cm langer Vlieslagenabschnitte erfordern würde, während das hier beschriebene Verfahren die Verwendung lediglich 100 cm langer Vlieslagenabschnitte erfordert.

Nach dem Hindurchführen durch den Spalt des Dehnwalzenpaares 15 kann es vorteilhaft sein, einer Einschnürung der Vliesverbundmaterialbahn 14 in Querrichtung Q entgegenzuwirken, welche Folge der Verkürzung der Bahn in Längsrichtung infolge der Rückstellkräfte der elastischen Filamente 6 sein kann. Hierzu kann ein weiteres Walzenpaar 31 dienen, welches die Bahn zumindest an dessen beiden Längsrändern fixiert. Vorteilhaft ist, der Einschnürung zumindest insoweit entgegenzuwirken, dass die Einschnürung, also die Verkürzung der Bahn in Querrichtung Q, also der Breite der Bahn, bezogen auf die Ausgangsbreite maximal 50%, insbesondere weniger als 30%, weiter insbesondere weniger als 20% und ganz besonders weniger als 10% beträgt.

Schließlich wird das Vliesverbundmaterial 24 im dargestellten Fall (Figur 1) auf eine Rolle 32 mit der Bahngeschwindigkeit v2 endlos aufgewickelt. Die Geschwindigkeit v2 wird hierbei vorzugsweise höher gewählt als die Geschwindigkeit v1, um die Bahn mit einer gewissen Vorspannung aufwickeln zu können. Denkbar und vorteilhaft wäre auch, die Bahn des Vliesverbundmaterials 24 direkt, also ohne vorheriges Aufwickeln auf die Rolle 31, der bestimmungsgemäßen Weiterverarbeitung, beispielsweise einer schnelllaufenden Maschine zur Herstellung pantförmiger Hygieneartikel zuzuführen. Auch solchenfalls würde dies mit einer Bahngeschwindigkeit erfolgen, die höher ist als die Geschwindigkeit v1.

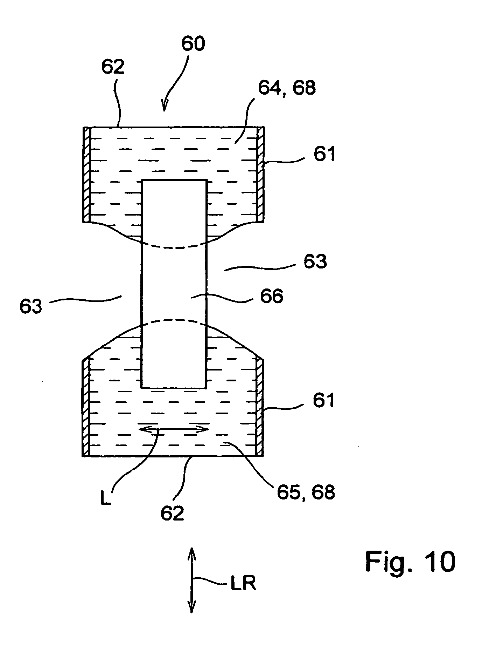

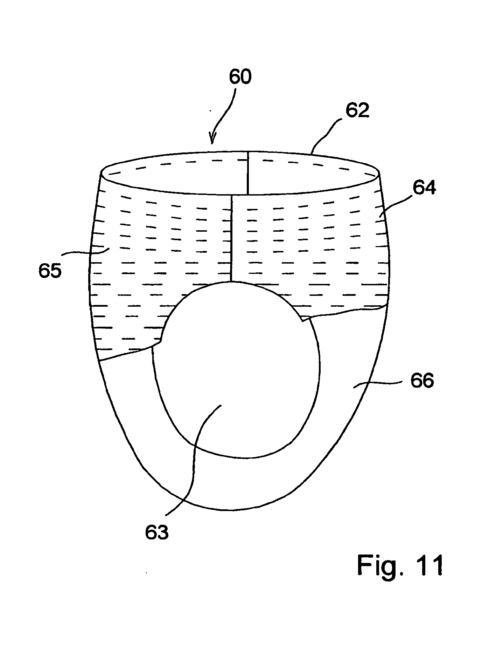

Schließlich zeigt Figur 10 einen wegwerfbaren absorbierenden Hygieneartikel 60 in Pantform jedoch im flachgelegten, ausgestreckten Zustand vor der herstellerseitigen Verbindung der Längsseitenrandabschnitte 61, mit einem eine Hüftöffnung bildenden in Umfangsrichtung nach der herstellerseitigen Verbindung der Längsseitenrandabschnitte 61 durchgehend geschlossenen Hüftrand 62 und mit Beinöffnungen 63, und mit Längsseitenrandabschnitte 61 aufweisendem Vorderteil 64 und Rückenteil 65. Vorderteil 64 und Rückenteil 65 sind voneinander in Längsrichtung LR des Hygieneartikels 60 beabstandet und werden in Längsrichtung LR des Hygieneartikels durch einen Absorptionsteil 66, der den Abstand zwischen Vorderteil 64 und Rückenteil 65 überbrückt verbunden. Figur 11 zeigt den wegwerfbaren absorbierenden Hygieneartikel 60 nach der herstellerseitigen Verbindung der Längsseitenrandabschnitte 61.

Das Absorptionsteil 66 ist auf der dem Körper zugewandten Seite des Vorder- und Rückenteils fixiert. Das Absorptionsteil 66 weist seinerseits ein flüssigkeitsundurchlässiges Backsheet, ein flüssigkeitsdurchlässiges Topsheet und einen dazwischen angeordneten flüssigkeitsaufnehmenden und -speichernden Absorptionskern auf. Der Absorptionskern enthält vorzugsweise superabsorbierende Polymerpartikel sowie Fasern, insbesondere natürliche Fasern, insbesondere in Form von Zellstofffluff.

Vorderteil 64 und Rückenteil 65 bestehen aus einem Vliesstoffverbundmaterialabschnitt 68, welcher jeweils aus einer Vliesstoffverbundmaterialbahn 24, hergestellt nach dem erfindungsgemäßen Verfahren, herausgetrennt, zum Beispiel herausgeschnitten oder herausgestanzt wurde, wobei die Längsrichtung L der Vliesstoffverbundmaterialbahn 24 im Wesentlichen senkrecht zu einer Längsrichtung LR des Hygieneartikels 60 verläuft. Die Erstreckung des Vorderteils 64 beträgt in Längsrichtung LR 20 cm, die des Rückenteils 65 beträgt in Längsrichtung LR 25 cm. Die Dehnbarkeit des Vorder- und Rückenteils in Umfangsrichtung der Hüftöffnung, also senkrecht zu einer Längsrichtung LR des Hygieneartikels 60 beträgt 100%, so dass der Umfang der Hüftöffnung verdoppelbar und damit einer Vielzahl von Konfektionsgrößen anpassbar ist.

**Patentansprüche**

1. Verfahren zur Herstellung einer Bahn eines elastischen Vliesstoffverbundmaterials (24) umfassend die folgenden Schritte:

   Zuführen und Fördern einer ersten Vliesstofflage (4, 4a) in einer Längsrichtung (L) als vorgefertigte Vliesstofflage, wobei die Vliesstofflage nach dem Spinnvlies-, Kardenvlies-, oder Spunlacevliesherstellverfahren hergestellt ist; Zuführen einer zweiten Vliesstofflage (10, 10a) als vorgefertigte Vliesstofflage, wobei die Vliesstofflage nach dem Spinnvlies-, Kardenvlies-, oder Spunlacevliesherstellverfahren hergestellt ist; Zuführen einer Vielzahl elastischer Filamente (6); Verbinden der Vielzahl elastischer Filamente (6) mit einer ersten Seite (70) der ersten Vliesstofflage (4, 4a) zu einem Materialverbund (14), wobei die elastischen Filamente (6) mit einem Spannungsfaktor von weniger als 1,3 mit der ersten Vliesstofflage (4, 4a) verbunden werden; wobei die zweite Vliesstofflage eine erste Seite (72) und eine zweite Seite (71) aufweist, und wobei die zweite Seite (71) der zweiten Vliesstofflage (10, 10a) mit einer ersten Seite (70) der ersten Vliesstofflage (4, 4a) verbunden wird, so dass die elastischen Filamente (6) sandwichartig zwischen der ersten Vliesstofflage (4, 4a) und zweiten Vliesstofflage (10, 10a) angeordnet werden; Zumindest bereichsweises Überdehnen der ersten und zweiten Vliesstofflage (4, 4a, 10, 10a) in der Längsrichtung (L) durch Hindurchführen des Materialverbunds (14) durch den Spalt eines Paars profilierter, ineinander greifender Dehnwalzen (15), wodurch der Materialverbund (14) in der Längsrichtung (L) gestreckt wird, wobei erste Bereiche (20) der ersten und zweiten Vliesstofflage (4, 4a, 10, 10a) stärker überdehnt werden als zweite Bereiche (21) der ersten und zweiten Vliesstofflage (4, 4a, 10, 10a), und wobei die ersten Bereiche (20) und zweiten Bereiche (21) der ersten und zweiten Vliesstofflage (4, 4a, 10, 10a) alternierend streifenförmig quer zu der Längsrichtung (L) angeordnet werden, und wobei die elastischen Filamente (6) in dem Vliesstoffverbundmaterial (24) derart angeordnet sind, dass sie im Wesentlichen in der Längsrichtung (L) orientiert sind, so dass sie höchstens 10 Schnittpunkte miteinander pro cm$^2$ aufweisen.

2. Verfahren nach Anspruch 1, **dadurch gekennzeichnet, dass** das Vliesstoffverbundmaterial (24) in der Längsrichtung (L) eine Dehnbarkeit von 35-300%, vorzugsweise von 50-200% aufweist.

3. Verfahren nach einem der Ansprüche 1-2, **dadurch gekennzeichnet, dass** die elastischen Filamente (6) direkt mit der ersten Vliesstofflage (4, 4a) verbunden werden, wobei die elastischen Filamente (6) gemeinsam mit der Vliesstofflage (4, 4a) durch einen Pressspalt (12), insbesondere einen beheizbaren Pressspalt (12) eines zweiten Walzenpaares, insbesondere eines Kalanderwalzenpaares (13) hindurchgeführt werden.

4. Verfahren nach einem der Ansprüche 1-3, **dadurch gekennzeichnet, dass** die Bahn des Vliesstoffverbundmaterials (24) nach dem Überdehnen der ersten Vliesstofflage (4, 4a) in seiner Quererstreckung fixiert wird, so dass einer Einschnürung der Bahn in Querrichtung (Q) entgegengewirkt wird, wobei die Einschnürung der Bahn des Vliesstoffverbundmaterials (24) vorzugsweise weniger als 50%, insbesondere weniger als 30%, weiter insbesondere weniger als 20% und weiter insbesondere weniger als 10% beträgt.

5. Verfahren nach einem der Ansprüche 1-4, **dadurch gekennzeichnet, dass** die elastischen Filamente mit einem Spannungsfaktor von weniger als 1,2, weiter vorzugsweise weniger als 1,1, weiter vorzugsweise weniger als 1,05 mit der ersten und/oder zweiten Vliesstofflage (4, 4a, 10, 10a) verbunden werden.

6. Verfahren nach einem der Ansprüche 1-5, **dadurch gekennzeichnet, dass** die zweiten Bereiche (21) im Wesentlichen nicht überdehnt werden.

7. Verfahren nach einem der Ansprüche 1-6, **dadurch gekennzeichnet, dass** der Materialverbund (14) beim Hindurchführen durch den Spalt eines Paars profilierter, ineinander greifender Dehnwalzen (15) um 35-300%, insbesondere um 50-200% gestreckt wird.

8. Verfahren nach einem der Ansprüche 1-7, **dadurch gekennzeichnet, dass** die Stärke der elastischen Filamente (6) 4-700 dtex, insbesondere 50-300 dtex, weiter insbesondere 100-200 dtex beträgt, wobei die elastischen Filamente (6) insbesondere ein thermoplastisches, insbesondere extrudierbares Polymer ausgewählt aus der Gruppe Polyurethane, elastische Polyester, elastische Polyamide, elastische Polyolefine, elastische Blockcopolymere, enthalten oder daraus bestehen.

9. Verfahren nach einem der Ansprüche 1-8, **dadurch gekennzeichnet, dass** die erste und/oder die zweite Vliesstofflage (4, 4a, 10, 10a) in der Längsrichtung (L) und/oder in der Querrichtung (Q) unelastisch ist.

**Claims**

1.  Method for producing a web of an elastic nonwoven composite material (24) comprising the following steps:

    feeding and delivering a first layer of nonwoven material (4, 4a) in a longitudinal direction (L) as a prefabricated layer of nonwoven material, the layer of nonwoven material being produced by the spunbonded, carded or spunlaced nonwoven production process;
    feeding a second layer of nonwoven material (10, 10a) as a prefabricated layer of nonwoven material, the layer of nonwoven material being produced by the spunbonded, carded or spunlaced nonwoven production process; feeding a multiplicity of elastic filaments (6); bonding the multiplicity of elastic filaments (6) to a first side (70) of the first layer of nonwoven material (4, 4a) to form a material composite (14), the elastic filaments (6) being bonded to the first layer of nonwoven material (4, 4a) with a tension factor of less than 1.3; the second layer of nonwoven material having a first side (72) and a second side (71), and the second side (71) of the second layer of nonwoven material (10, 10a) being bonded to a first side (70) of the first layer of nonwoven material (4, 4a), so that the elastic filaments (6) are arranged in a sandwich-like manner between the first layer of nonwoven material (4, 4a) and the second layer of nonwoven material (10, 10a); overstretching the first and second layers of nonwoven material (4, 4a, 10, 10a) in the longitudinal direction (L), at least in certain regions, by leading the material composite (14) through the gap between a pair of profiled, intermeshing stretching rolls (15), whereby the material composite (14) is stretched in the longitudinal direction (L), first regions (20) of the first and second layers of nonwoven material (4, 4a, 10, 10a) being overstretched more than second regions (21) of the first and second layers of nonwoven material (4, 4a, 10, 10a), and the first regions (20) and second regions (21) of the first and second layers of nonwoven material (4, 4a, 10, 10a) being arranged alternatingly in strip-like form transversely to the longitudinal direction (L), and the elastic filaments (6) being arranged in the nonwoven composite material (24) in such a way that they are oriented substantially in the longitudinal direction (L), so that they have at most 10 points of intersection with one another per cm$^2$.

2.  Method according to Claim 1, **characterized in that** the nonwoven composite material (24) has in the longitudinal direction (L) a stretchability of 35-300%, preferably of 500-200%.

3.  Method according to either of Claims 1 and 2, **characterized in that** the elastic filaments (6) are bonded directly to the first layer of nonwoven material (4, 4a), the elastic filaments (6) being led together with the layer of nonwoven material (4, 4a) through a pressing gap (12), in particular a heatable pressing gap (12), of a second pair of rolls, in particular a pair of calender rolls (13).

4.  Method according to one of Claims 1 to 3, **characterized in that**, after the overstretching of the first layer of nonwoven material (4, 4a), the web of nonwoven composite material (24) is fixed in its transverse extent, so that a constriction of the web in the transverse direction (Q) is counteracted, the constriction of the web of the nonwoven composite material (24) being preferably less than 50%, particularly less than 30%, more particularly less than 20% and more particularly less than 10%.

5.  Method according to one of Claims 1 to 4, **characterized in that** the elastic filaments are bonded to the first and/or second layer of nonwoven material (4, 4a, 10, 10a) with a tension factor of less than 1.2, more preferably less than 1.1, more preferably less than 1.05.

6.  Method according to one of Claims 1 to 5, **characterized in that** the second regions (21) are substantially not overstretched.

7.  Method according to one of Claims 1 to 6, **characterized in that**, as it is led through the gap of a pair of profiled, intermeshing stretching rolls (15), the material composite (14) is stretched by 35-300%, particularly by 50-200%.

8.  Method according to one of Claims 1 to 7, **characterized in that** the count of the elastic filaments (6) is 4-700 dtex, particularly 50-300 dtex, more particularly 100-200 dtex, the elastic filaments (6) particularly containing or consisting of a thermoplastic, particularly extrudable, polymer chosen from the group of polyurethanes, elastic polyesters, elastic polyamides, elastic polyolefins and elastic block polymers.

9. Method according to one of Claims 1 to 8, **characterized in that** the first and/or second layer of nonwoven material (4, 4a, 10, 10a) is unelastic in the longitudinal direction (L) and/or in the transverse direction (Q).

**Revendications**

1. Procédé de fabrication d'une bande d'un matériau composite non-tissé élastique (24), comprenant les étapes suivantes :

    l'introduction et le transport d'une première couche de non-tissé (4, 4a) dans une direction longitudinale (L) sous la forme d'une couche de non-tissé préfabriquée, la couche de non-tissé étant fabriquée selon le procédé de fabrication de non-tissés filés, de non-tissés cardés ou de non-tissés lacés par filage ; l'introduction d'une seconde couche de non-tissé (10, 10a) sous la forme d'une couche de non-tissé préfabriquée, la couche de non-tissé étant fabriquée selon le procédé de fabrication de non-tissés filés, de non-tissés cardés ou de non-tissés lacés par filage ; l'introduction d'une pluralité de filaments élastiques (6) ; la liaison de la pluralité de filaments élastiques (6) avec un premier côté (70) de la première couche de non-tissé (4, 4a) pour former un matériau composite (14), les filaments élastiques (6) étant reliés avec un facteur de tension inférieur à 1,3 avec la première couche de non-tissé (4, 4a) ; la seconde couche de non-tissé présentant un premier côté (72) et un second côté (71), et le second côté (71) de la seconde couche de non-tissé (10, 10a) étant relié avec un premier côté (70) de la première couche de non-tissé (4, 4a) de manière à ce que les filaments élastiques (6) soient placés en sandwich entre la première couche de non-tissé (4, 4a) et la seconde couche de non-tissé (10, 10a) ; le sur-étirement au moins dans des zones de la première et de la seconde couche de non-tissé (4, 4a, 10, 10a) dans la direction longitudinale (L) par passage du matériau composite (14) dans la fente d'une paire de cylindres d'étirement profilés (15), qui entrent en prise l'un avec l'autre, le matériau composite (14) étant ainsi étiré dans la direction longitudinale (L), des premières zones (20) de la première et de la seconde couche de non-tissé (4, 4a, 10, 10a) étant plus fortement sur-étirées que des secondes zones (21) de la première et de la seconde couche de non-tissé (4, 4a, 10, 10a), et les premières zones (20) et les secondes zones (21) de la première et de la seconde couche de non-tissé (4, 4a, 10, 10a) étant placées sous la forme de bandes alternées perpendiculairement à la direction longitudinale (L), et les filaments élastiques (6) étant placés dans le matériau composite non-tissé (24) de manière à être orientés essentiellement dans la direction longitudinale (L), de manière à présenter au plus 10 points d'intersection entre eux par cm$^2$.

2. Procédé selon la revendication 1, **caractérisé en ce que** le matériau composite non-tissé (24) présente une étirabilité de 35 à 300 %, de préférence de 50 à 200 %, dans la direction longitudinale (L).

3. Procédé selon l'une quelconque des revendications 1 à 2, **caractérisé en ce que** les filaments élastiques (6) sont reliés directement avec la première couche de non-tissé (4, 4a), les filaments élastiques (6) passant avec la couche de non-tissé (4, 4a) dans une fente de presse (12), notamment une fente de presse chauffable (12) d'une seconde paire de cylindres, notamment d'une paire de cylindres de calandre (13).

4. Procédé selon l'une quelconque des revendications 1 à 3, **caractérisé en ce que** la bande du matériau composite non-tissé (24) est fixée dans son étirement transversal après le sur-étirement de la première couche de non-tissé (4, 4a), de manière à entraver une contraction de la bande dans la direction transversale (Q), la contraction de la bande du matériau composite non-tissé (24) étant de préférence inférieure à 50 %, notamment inférieure à 30 %, plus particulièrement inférieure à 20 % et plus particulièrement inférieure à 10 %.

5. Procédé selon l'une quelconque des revendications 1 à 4, **caractérisé en ce que** les filaments élastiques sont reliés avec un facteur de tension inférieur à 1,2, de manière davantage préférée inférieur à 1,1, de manière davantage préférée inférieur à 1,05, avec la première et/ou la seconde couche de non-tissé (4, 4a, 10, 10a).

6. Procédé selon l'une quelconque des revendications 1 à 5, **caractérisé en ce que** les secondes zones (21) ne sont essentiellement pas sur-étirées.

7. Procédé selon l'une quelconque des revendications 1 à 6, **caractérisé en ce que** le matériau composite (14) est étiré de 35 à 300 %, notamment de 50 à 200 %, lors du passage dans la fente d'une paire de cylindres d'étirement profilés (15) qui entrent en prise l'un avec l'autre.

8. Procédé selon l'une quelconque des revendications 1 à 7, **caractérisé en ce que** l'épaisseur des filaments élastiques (6) est de 4 à 700 dtex, notamment de 50 à 300 dtex, plus particulièrement de 100 à 200 dtex, les filaments élastiques (6) contenant notamment un polymère thermoplastique, notamment extrudable, choisi dans le groupe des polyuréthanes, polyesters élastiques, polyamides élastiques, polyoléfines élastiques, copolymères séquencés élastiques, ou en étant constitués.

**9.** Procédé selon l'une quelconque des revendications 1 à 8, **caractérisé en ce que** la première et/ou la seconde couche de non-tissé (4, 4a, 10, 10a) est non élastique dans la direction longitudinale (L) et/ou dans la direction transversale (Q).

Fig. 1

7

8

Q

**Fig. 2**

48  4  6  49

10  50  51

**Fig. 3**

Fig. 4

Fig. 7

Fig. 8

Fig. 5

Fig. 6

15

Fig. 9 ( Stand der Technik )

EP 2 136 753 B1

Fig. 10

60

62

64

65

66

63

Fig. 11

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1184017 A1
- EP 1199058 A1
- EP 1308148 A2
- WO 0188245 A
- US 5219633 A
- US 4223063 A
- WO 06124092 A